Europäisches Patentamt

European Patent Office

Office européen des brevets

(19)

(11) Publication number: **0 460 569 A1**

# EUROPEAN PATENT APPLICATION

(12)

(21) Application number: **91109023.1**

(22) Date of filing: **03.06.91**

(51) Int. Cl.⁵: **C12Q 1/68**

(30) Priority: **04.06.90 IT 2052890**

(43) Date of publication of application:
**11.12.91 Bulletin 91/50**

(84) Designated Contracting States:
**AT BE CH DE DK ES FR GB GR IT LI LU NL SE**

(71) Applicant: **TECNOGEN S.C.P.A.**
**Località "La Fagianeria-P.CO SCIENTIFICO"**
**I-81015 Piana de Monte Verna, (Caserta)(IT)**

(72) Inventor: **CORTI, ANGELO**
**VIA ROSSINI, 14**
**IT-24052 AZZANO S.P. BERGAMO(IT)**

(74) Representative: **Gervasi, Gemma, Dr. et al**
**NOTARBARTOLO & GERVASI Srl 33, Viale**
**Bianca Maria**
**I-20122 Milano(IT)**

(54) **PROCESS FOR IDENTIFYING POLYNUCLEOTIDE SEQUENCES AND DEVICE SUITABLE THEREFOR.**

(57) There is described a process for identifying polynucleotide sequences consisting in hybridizing in one single capsule polynucleotide molecules which comprise said sequences with at least one reagent, also of polynucleotide nature, having sequence complementary to the polynucleotide to be detected.

The process herein disclosed is exceptionally simple and it can be carried out also in unspecialized laboratories by means of a kit.

EP 0 460 569 A1

A. Technical field.

The present invention relates to a process for identifying polynucleotide sequences consisting in hybridizing in one single capsule polynucleotide molecules which comprise said sequences with at least one reagent, also of polynucleotide nature, having sequence complementary to that of the polynucleotide to be detected. Typically the process of the invention is carried out by making use of a kit comprising all reagents and instructions necessary for the process to be run also by unskilled practitioner and outside very equipped and specialized laboratories. The hybridisation assays are known to be based on the use of at least one analytical reagent of polynucleotide type, hereinafter designated as probe, having sequence complementary to the sequence of the analyte polynucleotide or target. The presence of the polynucleotide target in the test is put in evidence by the hybridisation reaction with the polynucleotide probe. The application in the clinical diagnosis of hybridisation assays based on nucleic acid probes are widely documented in literature (Matthews J. A. and Kricka L., Analytical Biochemistry, 169, 1-25, 1989). The hybridisation assays of polynucleotide sequences are not only powerful tools for the basic research, but they are turning out to be potentially useful tools also in many other practical fields such as the prenatal diagnosis of genetic diseases performed on specimens obtained from amniocentesis or from tests of peripheral blood, the identification of subjects from medical-legal exhibits and the identification of pathogen agents comprised in biological specimens of human, animal or plant origin or comprised in foodstuff and in the environment. On this point it is important to stress that the attainable levels of sensitivity and specificity allow the identification of bacterial and viral pathogenic agents in amounts not detectable by the traditional immunoassays.

B. Background of the invention.

Despite of the undoubted importance of the nucleic acid hybridisation assays in the domain of the clinical, veterinary and environmental diagnosis, their application is still limited. The reason thereof mainly depends on the technical difficulties in performing the hybridisation assays, which prevent their use in less specialized laboratories.

Many techniques have been described in literature, each exhibiting advantages or drawbacks concerning sensitivity, specificity, ease of execution, quickness and safety for the practitioner and for the environment. In detail there have been widely described techniques concerning:
a) the preparation of the analytical test (extraction, purification and amplification of the target nucleotide);
b) labelling strategy of the probe necessary to reveal the hybridisation reaction;
c) hybridisation strategy. In particular a complete description of said strategies is reported by Matthews J. A. and Kricka L., Analytical Chemistry, 169, 1-25 (1989).

Most of the known techniques are based on the use of heterogeneous analytical systems where the target polynucleotide is immobilized on solid support in order to enable the hybridized and nonhybridized probe separation.

The most serious drawback of this technique is that the analysis is very time consuming because of the extremely slow hybridisation kinetics and complex operations for the separation of hybridized and nonhybridized probe. Said drawback was partially overcome upon development of hybridisation techniques in homogeneous phase, which are based on much quicker hybridisation reactions. Nevertheless the remarkable difficulties due to the necessity of specific equipments for monitoring the "bound/free" ratio did not allow so far the widespread use of this type of technique. The poor diffusion of the hybridisation methods is specifically felt in the field of infectivity, where on the contrary the quite easier immunoassays are usually applied, which moreover allow the reading of the results in the visible.

The recent disclosure of the DNA labelling system by polymerase chain reaction (PCR) (Saiki et al. Science, 230, 1350 (1985)) has dramatically reduced the need of developing high sensitive assays since a few copies of the target polynucleotide can be amplified thousands of times. Nevertheless the need still exists of simple and quick developed systems enabling the amplified product to be reliably identified through hybridisation reactions with specific probes.

C. The technical problem.

It is evident from the aforementioned arguments how difficult is the exploitation of hybridisation techniques in a situation other than that of specialized laboratories and with highly skilled practitioner. The basic problem to be solved is therefore to provide to anyone who is concerned, a simple and reliable method, which does not require expensive equipments or equipments difficult to be used outside a laboratory, as already developed for instance in the immunological field.

The problem is still more serious because of the known fact that the assay sensitivity depends on the hybridisation time and on the probe concentration and therefore it normally needs a suitable milieu such as a well equipped laboratory.

D. Detailed description of the invention.

It has now been discovered that the hybridisation of complementary polynucleotide partners can be achieved, in that one member, unlabeled, of the pair is immobilized on solid phase, whereas the second, labeled with hapten groups, is freely spread in the solution and making use of one single reaction space in which all the steps of the identification process are subsequently carried out.

In particular the process for the identification of polynucleotide sequences according to the invention consists in performing the essential steps within one single capsule enclosing a microporous membrane overlapping a cylinder of adsorbent stuff and solid with said membrane; said capsule having the upper rims slightly prominent over said membrane to delimitate a small well for the loaded liquids. Said essential steps are the following:

a) loading on the membrane a solution of the target unlabeled polynucleotide molecules intended to be identified in such a way to immobilize said molecules onto said membrane;

b) hybridizing at temperature comprised from 20°C to 70°C and for a time comprised from 5 minutes to 24 hours the polynucleotide molecules fixed to said membrane with a solution of labeled polynucleotide probes;

c) washing the membrane with one or more usual buffer solutions in order to remove therefrom the unbound labeled polynucleotide probe;

d) developing a colorimetric reaction with the solution of an indicator substance reacting with the hybridized polynucleotide molecules of step b).

In any case the unique reaction vessel in which all the stages of the process of the invention are carried out shows the following essential features:

a) a layer of microporous material of thickness comprised from 0.1 to 0.3 mm essentially intended: - to immobilize the unlabeled polynucleotide molecules to be hybridized; - to function as hybridisation space for labeled and unlabeled polynucleotides; - to allow the reading of the results after development of the colorimetric reaction with the indicator substance for hybridized molecules;

b) a layer of adsorbent material below the microporous one and in close contact thereto, having a total volume comprised from 2 to 10 cm$^3$, capable of adsorbing all the solutions applied for executing the process of the invention (hybridisation, washing and detection solutions) and of maintaining the microporous layer sufficiently impregnated with said solutions, thus allowing long hybridisation kinetics and high concentrations of the probe;

c) a solid vessel (capsule) enclosing the two layers a) and b) and preventing whatever release in the environment of the materials used in the process of the invention.

Typically the practical embodiment of the process of the invention provides the sequential loading, adsorption and incubation within said unique reaction space of:

a) a solution comprising the unlabeled polynucleotide;

b) a pre-hybridisation solution;

c) a solution comprising the polynucleotide preferably labeled with hapten groups;

d) washing solutions for the bound/free separation;

e) solutions comprising molecules able to specifically bound the hapten groups conjugated to the hybridized polynucleotide, said molecules being suitably labeled with systems for the visual detection of the reaction area within the microporous layer of the reaction capsule.

The process according to the invention enable the polynucleotide sequences present in the material under test to be easily identified by merely depositing the different solutions upon the microporous part of the reaction capsule and incubating for a suitable time varying from a few minutes to many hours depending on the required sensitivity, without the need of complex equipments and procedures. It is an important feature of the present invention the fact that the hybridisation reaction may unpredictable continue for several hours, even after the complete adsorption of the labeled polynucleotide solution within the microporous layer of the reaction capsule. It is submitted, without however being bound to theory somewhere limiting the present invention, that the presence of the adsorbing layer below, which is swollen with the pre-hybridisation solution, contributes to maintain the humidity within the reaction space, thus facilitating the molecule mobility and allowing the hybridisation reaction to run for times otherwise only achievable with more sophisticated systems based on the use of membranes completely dipped in the hybridisation solution.

The present invention thus allows high sensitivity to be achieved owing to the possibility of extending the hybridisation times, without however disadvantaging the ease of the assay execution.

The present process allows furthermore the use of reduced volumes of reagents and labeled polynucleotides in higher concentration. This feature not only brings about an increase in assay sensitivity but it is particularly desirable for the manufacture of kits.

Among all the materials suitable for the preparation of the microporous layer of the reaction capsule, nitrocellulose or nylon are referred to. The porosity of said materials is preferably comprised from 0.4 to 1.2 $\mu$m. Among the materials suitable for the preparation of the adsorbent layer of the reaction vessel, hydrophilic stuff, particularly cotton-wool and adsorbent or filter paper are considered.

Commercially available devices normally employed to perform immunotests may also be used. As already said above, well known and previously extensively disclosed techniques for labeling polynucleotide sequences with haptenes may be applied (Matthews J.A. and Kircka L., Analytical Biochemistry, 169, 1-25, 1989). Also known are the nonradioactive labeling and detecting systems for nucleic acids which include the direct labeling with enzymes (peroxidase, alkaline phosphatase, etc.) or the indirect labeling with biotin (primary label) which can be revealed with secondary labels such as avidin, streptavidin and anti-biotin antibodies conjugated to enzymes (alkaline phosphatase, $\beta$-galactoxidase, glucose oxidase, peroxidase and biotinyl-enzyme/avidin complexes) or conjugated to colloidal metals, which can be easily exploited in the process of the invention. Other haptens usually employed as labeling agents for polynucleotide sequences include digoxigenin, sulphonic groups, dinitrophenyl groups, N-2-acetyl-aminofluorene, N-2-acetyl-amino-7-iodofluorene, ethidium and 5-bromodeoxyuridine, which can be detected by anti-hapten antibodies directly or indirectly labeled as for the anti-biotin antibodies.

As a further option, detection systems based on the polynucleotide primary labeling with glycosyl-groups revealed with enzyme-conjugated Concanavalin-A or with poly(dA) revealed with peroxidase conjugated poly (dT) are described.

A further reagent, necessary by using enzyme systems to evidence the hybridisation reactions, consists of an insoluble enzymatic substrate, which can settle on the microporous layer in the reaction capsule, without giving rise to diffusion.

Examples of insoluble substrates for alkaline phosphatase or peroxidase comprise bromo-chloro-indolyl phosphate/nitro blue tetrazolium (BICP/NBT), 4-chloro-1-naphthol, 3-amino-9-ethylcarbazole and diaminobenzidine. A preferred embodiment of the present invention provides the immobilisation of the polynucleotide analyte onto the nitrocellulose microporous layer of the reaction capsule, followed by direct hybridisation with the polynucleotide probe, either biotinylated or conjugated to digoxigenin, diluted in a buffer suitable for hybridisation on nitrocellulose membranes, which buffer comprises for instance sodium citrate, formamide, N-laurylsarcosine, sodium dodecylsulphate, casein etc. After accomplishment of the hybridisation reaction, the detection is carried out with streptavidin or with alkaline phosphatase-labeled anti-digoxigenin antibodies and following enzymatic reaction with BICP/NBT. Said preferred embodiment of the invention avails the possibility of analysing different polynucleotide analytes making use of one single microporous layer of the reaction vessel, since different samples can be immobilized in form of small spots of 1-2 mm diameter on the surface of the microporous layer, which is 1-2 cm in diameter. Furthermore said preferred embodiment of the invention allows positive and negative controls (labeled polynucleotides and polynucleotides non-homologous to the probe, immobilized onto the microporous layer of the reaction capsule) to be added, thus allowing the simultaneous control of the reagents and of the assay development.

Another preferred embodiment of the process of the invention is based on the immobilisation of the polynucleotide probe on the microporous layer of the reaction capsule and on the following hybridisation in liquid phase (reverse hybridisation) to the polynucleotide analyte enzymatically labeled by amplification with polymerase chain reaction (PCR) in presence of dUTP-biotin or dTTP-digoxigenin. By this way the polynucleotide analyte is at the same time amplified and labeled with hapten groups and can be easily detected with the method of the present invention. Still in this case the further advantage is provided that several polynucleotide probes, for instance specific for different viral pathogenic agents likely present in the test, may be immobilized and that the target polynucleotide may hybridise with PCR amplified and labeled probes, in one single assay, comprising negative and positive controls. According to a still more preferred embodiment of the present invention all materials and reagents necessary for performing the process are comprised in one single kit, which allows said process to be easily carried out also outside well equipped laboratories.

In particular the kit consists of the following essential parts:

a) one capsule enclosing a microporous membrane overlapping a cylinder of adsorbent stuff and solid with said membrane, said capsule having the upper rims slightly prominent over said membrane to delimitate a small well for the loaded liquids;

b) a vial containing a solution of labeled polynucleotide probe molecules;

c) three vials containing the necessary reagents for preparing a buffer solution selected from the group consisting of 5xSSC, pH 7.0, comprising 50% (v/v) formamide (Fluka), 0.1% (w/v) N-lauryl-sarcosine, 0.02% (w/v) sodium dodecylsulphate, 5% (w/v) blocking reagent 1096176 (Böhringer Mannheim), 10 μg/ml salmon or herring sperm DNA;

d) a vial containing a solution of a revealing substance consisting of alkaline phosphatase -conjugated anti-digoxigenin antibodies;

e) two vials containing the alkaline phosphatase substrate BICP/NBT;

f) a micropipette of 100-400 μl;

g) an instruction leaflet disclosing the protocol for the drawing of the samples to analyse and for the operations to be performed with the different kit constituents to identify the polynucleotide sequences in the samples under test.

EXAMPLE 1.

Determination of DNA sequences from plasmid pBR328. The following solutions have been deposited by means of normal micropipettes upon the surface of the microporous layer of the reaction capsule consisting of an adsorbent filter commercialized under the Trade Mark Porex(R) Format System by Porex Technology, Inc. Fairburn, GA 30213, adsorbed and incubated as hereinafter reported:

a) distilled water, 100 μl (30 seconds at room temperature (RT));

b) 3 M sodium chloride, 0.3 M sodium citrate, pH 7.0 (20xSSC), 100 μl (5 min. at RT);

c) target polynucleotide (DNA from plasmid pBR328 denatured at 100°C for 10 minutes, diluted to different concentrations with herring or salmon sperm DNA solution 50 ng/ml);

d) pre-hybridisation solution filtered on 0.22 μm filter (5xSSC, pH 7.0 comprising 50% (v/v) formamide (Fluka), 0.1% (w/v) N-lauryl-sarcosine, 0.02% (w/v) sodium dodecylsulphate, 5% (w/v) blocking reagent 1096176 (Böhringer Mannheim), 400 ml, (4 minutes at 42°C); e) polynucleotide probe (pBR328 DNA labeled with digoxigenin (Böhringer Mannheim) diluted to 100 μl/ml in prehybridisation solution comprising 10 μg/ml herring or salmon sperm DNA), 100 μl, (15 minutes to 6 hours depending on the necessary sensibility, at 42°C). At this stage the Porex(R) filters are wrapped in Parafilm(R);

f) washing solution (0.1xSSC comprising sodium dodecyl sulphate 0.1% (w/v)), 100μl, (four times, 30 seconds for wash, at 42°C);

g) washing solution (10 mM Tris HCl, 150 mM NaCl pH 7.5, (TBS)), 100 μl, (30 seconds at RT );

h) Blocking reagent 1096176 0.5% (w/v) (Böhringer Mannheim), 200 μl, (2 minutes at RT);

i) alkaline phosphatase conjugated anti-digoxigenin antibody (Böhringer Mannheim), 1:200 in TBS, 100 μl (4 minutes at RT);

l) washing solution (TBS), 100 μl (three times, 30 seconds each at RT);

m) washing solution (Tris HCl 10 mM, NaCl 100 mM, MgCl2, pH 9.5), 100 μl (30 seconds at RT);

n) Alkaline phosphatase chromogenic substrate BICP/NBP (Biorad kit), 400 μl ( 1 hour at RT).

The results obtained in form of grey-bluish stains on a clear background have been visually evaluated and reported in table 1. As it can be appreciated, detectable stains have been obtained with amounts of pBR328 homologous DNA (target polynucleotide) ranging from 10 pg to 4 ng depending on the hybridisation time according to the model of the present invention. No stains was observed with DNA from herring or salmon sperm (nonhomologous homologous DNA) even in definitively higher amounts (50 ng), thus suggesting that homologous DNA strands only give rise to hybridisation during the assay.

TABLE 1- Determination of plasmid pBR328 DNA with the technique of the present invention at different hybridisation times.

| Hybridisation time | pBR 328 (pg) | signal intensity (1) |
|---|---|---|
| 15 minutes | 4000 | +++ |
| | 200 | + |
| | 10 | - |
| | 0.5 | - |
| | 0 | - |
| 1 hour | 4000 | ++++ |
| | 200 | ++ |
| | 10 | +/- |
| | 0.5 | - |
| | 0 | - |
| 2 hours | 4000 | +++++ |
| | 200 | +++ |
| | 10 | + |
| | 0.5 | - |
| | 0 | - |
| 6 hours | 4000 | +++++++ |
| | 200 | +++++ |
| | 10 | ++ |
| | 0.5 | +/- |
| | 0 | - |

1) visually evaluated.

EXAMPLE 2

Determination of Alu-1 sequences in genomic DNA samples extracted from human-mouse somatic hybrids. Genomic DNA tests (1 $\mu$g/$\mu$l) have been extracted from human cells, murine cells and somatic human-murine hybrids. 1 µl sample aliquots have been seeded upon and immobilized onto Porex(R) Format System filters (Porex Technologies, Inc. Fairburn, GA 30213) and analysed by hybridisation by means of a polynucleotide probe Alu-1-digoxigenin (hybridisation time 4 hours) as disclosed in example 1. The Alu-1 probe (0.3 Kb) had been previously labeled with digoxigenin by means of the kit "DNA labeling and detection Nonradioactive" (Böhringer Mannheim ) according to the manufacturer instructions.

The obtained results are reported in Table 2. As it can be appreciated DNA from cells only, which contain human chromosomes (i.e. human cells and human-murine hybrids) results to be positive to the assay, as otherwise expected since the tandem Alu-1 sequences are known to be typical of the human genome. Thus the assay specificity is further confirmed by the absence of reaction with DNA from murine cell. Table 2 - Determination of Alu-1 sequences within the genome DNA of different cell lines by the technique of the present invention.

| Cell line | human chromosomes | Alu-1 sequences |
|---|---|---|
| MOLT4 (human) | all | +++ |
| LMTK- (murine) | 0 | - |
| 5468F1c16 (hybrid) | 1,2,6,8,10,11,12, 13,14,17,21,22 | ++ |
| 5868F1c15 (hybrid) | 2,12,17,20 | + |

## Claims

1. Process for the identification of polynucleotide sequences consisting in performing the following essential steps within one single capsule enclosing a microporous membrane overlapping a cylinder of adsorbent stuff and solid with said membrane, said capsule having the upper rims slightly prominent over said membrane to delimitate a small well for the loaded liquids, the steps being: a) loading a solution of the target unlabeled polynucleotide molecules intended to be identified upon the membrane in such a way to immobilize said molecules onto said membrane;
b) hybridizing at temperature comprised from 20°C to 70°C and for a time comprised from 5 minutes to 24 hours the polynucleotide molecules fixed to said membrane with a solution of labeled polynucleotide probes;
c) washing the membrane with one or more usual buffer solutions in order to remove therefrom the unbound labeled polynucleotide probe; d) running a colorimetric reaction with the solution of an indicator substance reacting with the hybridized polynucleotide molecules of step b).

2. Process according to claim 1, characterized in that the labeling of said polynucleotide probe molecules is directly obtained with enzymes.

3. Process according to claim 1, characterized in that the labeling of said polynucleotide probe molecules is directly obtained with peroxidase or alkaline phosphatase.

4. Process according to claim 1, characterized in that the labeling of said polynucleotide probe molecules is directly obtained by chemical or enzymatic modification with hapten groups or biotin.

5. Process according to claim 1, characterized in that the labeling of said polynucleotide probe molecules is indirectly obtained by chemical or enzymatic modification with hapten groups or biotin and the primary label is revealed with avidin, streptavidin, anti-biotin antibodies, each conjugated with enzymes selected from the group consisting of alkaline phosphatase, $\beta$-galactosidase, glucose oxidase, perox- idase, biotinyl-enzyme/ avidin complexes or colloidal metal-conjugated anti biotin antibodies.

6. Process for the identification of polynucleotide sequence consisting in performing the following essential steps within one single capsule enclosing a microporous membrane overlapping a cylinder of adsorbent stuff and solid with said membrane, said capsule having the upper rims slightly prominent over said membrane to delimitate a well for the loaded liquids, the steps being:

a) loading a solution of the unlabeled polynucleotide probe molecules used to hybridize the polynucleotide target molecules intended to be identified upon the membrane in such a way to immobilize said molecules onto said membrane;

b) hybridizing at temperature comprised from 20°C to 70°C and for a time comprised from 5 minutes to 24 hours the membrane fixed polynucleotide probe molecules with a solution of labeled polynucleotide target molecules;

c) washing the membrane with one or more usual buffer solutions in order to remove therefrom the unbound labeled polynucleotide target; d) running a colorimetric reaction with the solution of an indicator substance reacting with the hybridized polynucleotide molecules of step b).

7. Process according to claim 6, characterized in that the labeling of said polynucleotide target molecules is obtained by polymerase chain reaction (PCR).

8. Process according to claim 6, characterized in that the labeling of said polynucleotide target molecules is obtained by incorporation of nucleotides modified with hapten groups or biotin.

9. Process according to claim 6, characterized in that the labeling of said polynucleotide target molecules is obtained by incorporation of nucleotides modified with hapten groups or biotin and the primary label is revealed with avidin, streptavidin, anti-biotin antibodies, each conjugated with enzymes selected from the group consisting of alkaline phosphatase, $\beta$-galactosidase, glucose oxidase, peroxidase, biotinyl-enzyme/ avidin complexes or colloidal metal-conjugated anti biotin antibodies.

10. Kit for identifying polynucleotide sequences consisting of:

a) one capsule enclosing a microporous membrane overlapping a cylinder of adsorbent stuff and solid with said membrane, said capsule having the upper rims slightly prominent over said membrane to delimitate a small well for the deposited liquids;

b) a vial containing a solution of labeled polynucleotide probe molecules;

c) three vials containing the necessary reagents for preparing a buffer solution selected from the group consisting of 5xSSC, pH 7.0, comprising 50% (v/v) formamide (Fluka), 0.1% (w/v) N-lauryl-sarcosine, 0.02% (w/v) sodium dodecylsulphate, 5% (w/v) blocking reagent 1096176 (Böhringer Mannheim), 10 $\mu$g/ml salmon or herring sperm DNA;

d) a vial containing a solution of a revealing substance consisting of alkaline phosphatase-conjugated anti-digoxigenin antibodies;

e) two vials containing the alkaline phosphatase substrate BICP/NBT;

f) a micropipette of 100-400 $\mu$l;

g) an instruction leaflet disclosing the protocol for the drawing of the samples to analyse and for the operations to be run with the different kit constituents to identify the polynucleotide sequences in the samples under test.

11. Kit for identifying polynucleotide sequences consisting of:

a) one capsule enclosing a microporous membrane overlapping a cylinder of adsorbent stuff and solid with said membrane, said capsule having the upper rims slightly prominent over said membrane to delimitate a small well for the deposited liquids and already bearing the unlabeled probe and control polynucleotides and the labeled control polynucleotides;

b) a vial containing a solution of nucleotides labeled with haptens by amplification and labeling of polynucleotide sequences means PCR;

c) three vials containing the necessary reagents for preparing a buffer solution selected from the group consisting of 5xSSC, pH 7.0, comprising 50% (v/v) formamide (Fluka), 0.1% (w/v) N-lauryl-sarcosine, 0.02% (w/v) sodium dodecylsulphate, 5% (w/v) blocking reagent 1096176 (Böhringer Mannheim), 10 $\mu$g/ml salmon or herring sperm DNA;

d) a vial containing a solution of a revealing substance consisting of alkaline phosphatase-conjugated anti-digoxigenin antibodies;

e) two vials containing the alkaline phosphatase substrate BICP/NBT;

f) a micropipette of 100-400 $\mu$l;

8

g) an instruction leaflet disclosing the protocol for the drawing of the samples to analyse and for the operations to be run with the different kit constituents to identify the polynucleotide sequences in the samples under test.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 253 578 (HYBRITECH INC.) <br> * Column 4, line 15 - column 5, line 16; column 11, lines 44-56; column 13, lines 34-56; claims 1-3,11,15,16 * | 1,10 | C 12 Q 1/68 |
| Y | | 6 | |
| Y | EP-A-0 281 927 (MOLECULAR DIAGNOSTICS, INC.) <br> * Abstract; page 3, lines 45-57; claims * | 6 | |
| A | EP-A-0 322 311 (APPLIED BIOSYSTEMS, INC.) <br> * Page 3, lines 37-45; page 4, line 46 - page 5, line 7; page 7, line 25 - page 9, line 30 * | 8,9 | |
| A | WO-A-8 505 451 (HYBRITECH INC.) <br> * Abstract; page 3, line 5 - page 4, line 31; page 5, lines 16-25; page 12, line 30 - page 13, line 2; claims 1-7,21-28 * | 1,6 | |
| A | EP-A-0 311 388 (ORTHO DIAGNOSTIC SYSTEMS) <br> * Page 3, line 15 - page 7, line 10 * | 1 | |
| A | NUCLEIC ACIDS RESEARCH, vol. 16, no. 17, 1988, page 8719, IRL Press Ltd, Oxford, GB; Y.-M.D. LO et al.: "Rapid production of vector-free biotinylated probes using the polymerase chain reaction" <br> * The whole document * | 6-9 | TECHNICAL FIELDS SEARCHED (Int. Cl.5) <br><br> C 12 Q <br> G 01 N |
| P,Y | EP-A-0 420 260 (F. HOFFMANN-LA ROCHE) <br> * Abstract; page 4, lines 42-46; page 9, line 50 - page 10, line 9; page 10, lines 50-58 * | 6 | |

The present search report has been drawn up for all claims

| Place of search | Date of completion of search | Examiner |
|---|---|---|
| The Hague | 20 September 91 | LUZZATTO E.R.P.G.A. |

CATEGORY OF CITED DOCUMENTS
X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document